# EUROPEAN PATENT SPECIFICATION

(11) **EP 3 865 155 B1**
(45) Date of publication and mention of the grant of the patent: **30.11.2022**
(21) Application number: 20305138.8
(22) Date of filing: 13.02.2020
(51) Int. Cl.: A61K 51/10, A61P 35/00

(54) **PROCESS FOR SITE-SPECIFIC MODIFICATION OF AN ANTIBODY**
VERFAHREN ZUR STELLENSPEZIFISCHEN MODIFIZIERUNG EINES ANTIKÖRPERS
PROCÉDÉ DE MODIFICATION D'UN ANTICORPS SPÉCIFIQUE AU SITE

(43) Date of publication of application: 18.08.2021
(73) Proprietor: Orano Med, 92320 Chatillon (FR)
(72) Inventor: A. STALLONS, Tania, WYLIE, TX 75098 (US); TORGUE, Julien, GAITHERSBURG, MD 20878 (US); E. KIEFER, Garry, RICHARDSON, TX 75082 (US); ROJAS-QUIJANO, Federico, BEDFORD, TX 76021 (US)
(74) Representative: Lavoix

(56) References cited:
- SIMONE JEGER ET AL: "Site-Specific and stoichiometric Modification of Antibodies by Bacterial Transglutaminase", ANGEWANDTE CHEMIE INT ED, WILEY-VCH, DE , vol. 49, no. 51 17 December 2010 (2010-12-17), pages 9995-9997, XP002695029, ISSN: 1433-7851, DOI: 10.1002/ANIE.201004243 Retrieved from the Internet: URL:http://onlinelibrary.wiley.com/doi/10. 1002/anie.201004243/pdf [retrieved on 2010-11-25] -& SIMONE JEGER ET AL: "Site-Specific and Stoichiometric Modification of Antibodies by Bacterial Transglutaminase - SUPPORTING INFORMATION", ANGEWANDTE CHEMIE INTERNATIONAL EDITION, vol. 49, no. 51, 17 December 2010 (2010-12-17), pages e1-e46, XP055080950, DOI: 10.1002/anie.201004243
- JÜRGEN GRÜNBERG ET AL: "DOTA-Functionalized Polylysine: A High Number of DOTA Chelates Positively Influences the Biodistribution of Enzymatic Conjugated Anti-Tumor Antibody chCE7agl", PLOS ONE, vol. 8, no. 4, 2 April 2013 (2013-04-02), page e60350, XP055104572, ISSN: 1932-6203, DOI: 10.1371/journal.pone.0060350
- SCHNEIDER HENDRIK ET AL: "Recent progress in transglutaminase-mediated assembly of antibody-drug conjugates", ANALYTICAL BIOCHEMISTRY, ACADEMIC PRESS, AMSTERDAM, NL, vol. 595, 5 February 2020 (2020-02-05), XP086076204, ISSN: 0003-2697, DOI: 10.1016/J.AB.2020.113615 [retrieved on 2020-02-05]

## Description

The present invention concerns the field of nuclear medicine in particular for diagnosing or treating cancer.

For the delivery of medically interesting ions or radionuclides, appropriate chelating agents forming stable complexes are of fundamental importance. Additionally, chelating agents can be conjugated to monoclonal antibodies to achieve targeted delivery to the cancer cells. The constructs as produced (so called bioconjugated antibodies, antibodypayload conjugates, or antibody-drug conjugates (ADC)) find increased interest for the targeted treatment of cancer. The modified antibody can then accumulate in the tumour cells, concentrating the effects of the radioactivity of the radionuclide.

ADCs have gained FDA-approval (Adcetris, Kadcyla, Besponsa and Mylotarg) all of which have their payload chemically attached to the antibody in a non-site specific manner. Hence, the resulting product is highly heterogeneous, both with respect to the stoichiometric relationship between antibody and payload (payload antibody ratio, or drug to antibody ratio, or DAR), as well concerning the conjugation sites on the antibody. Each of the resulting species, although in the same drug product, may have distinct properties that could potentially lead to a wide range of different *in-vivo* pharmacokinetic properties and activities.

Studies have shown that a site-specific drug attachment can lead to a significantly higher tumor uptake and a decreased uptake in non-targeted tissues compared to the non-site specific ADC. These data suggest that stoichiometrically well-defined ADCs display improved pharmacokinetics and better therapeutic indexes compared to chemically modified ADCs.

As a site-specific technology, enzymatic conjugation has shown great interest since these conjugation reactions are typically fast and can be done under physiological conditions. Among the available enzymes, microbial transglutaminase (MTG) from the species *Streptomyces mobaraensis* has found increasing interest as an attractive alternative to conventional chemical protein conjugation of functional moieties including antibodies. The MTG catalyzes under physiological conditions a transamidation reaction between a 'reactive' glutamine of a protein or peptide and a 'reactive' lysine residue of a protein or peptide, whereas the latter can also be a simple, low molecular weight primary amine such as a 5- aminopentyl group.

Jeger et al. (Angew. Chem. Int. Ed. 2010, 49, 9995-9997) and Dennler et al. (Bioconjugate Chemistry 2014, 25, 569-578) report that only few glutamines are typically targeted by MTG, thus making the MTG an attractive tool for site-specific and stoichiometric protein modifications. Glutamine 295 (Q295) was identified as the only reactive glutamine on the heavy chain of different IgG types to be specifically targeted by MTG with low-molecular weight primary amine substrates (Jeger et al.).

It has been shown by Dennler *et al.* that quantitative conjugation to Q295 was only possible upon removal of the glycan moiety at the asparagine residue 297 (N297) with PNGase F, while glycosylated antibodies could not be conjugated efficiently.

WO 2017/025179 discloses a protein site-specific bioconjugation method to produce a protein comprising at least one acyl glutamine-containing amino donor sequence covalently linked via a γ-glutamyl-amide bond to an amino donor-comprising substrate, wherein the at least one acyl glutamine-containing amino acid donor sequence comprises at least the amino acid sequence TYFQAYG. The method of WO 2017/025179 therefore requires beforehand a cumbersome engineering process upon the protein in order to attach it the acyl glutamine-containing amino acid tag.

It thus is still desirable to provide a simple and efficient method to achieve antibodies bioconjugated with chelating agents with a defined DAR and in a site specific fashion.

The present invention is directed to a method for site-specifically conjugating antibodies to chelators in a way that would produce a defined Chelator-to-antibody ratio (CAR), said method being robust and easily implementable.

This method does not involve genetically engineering, growing and purifying each antibody of interest, which is common in many bioconjugation protocols. Instead this method allows the use of any antibody to bioconjuate to a chelator.

According to an object, the invention concerns a process for preparing a site-specific bioconjugated antibody of formula (I):

Ab-(Linker-Chelator)ₙ (I)

where
Ab is an antibody,
Linker is an oligopeptide with a COOH terminal end and a N-terminal end, Chelator is a metal chelating agent,
n is the Chelator-to-antibody ratio (CAR), wherein 0<n≤2;
said process comprising :
   - the enzymatic deglycosylation of said antibody ;
   - the coupling of the obtained deglycosylated antibody with a compound of formula (A):

      Linker-Chelator (A)
in the presence of a transglutaminase,
said process being characterized in that:
   said Linker is bound to Ab at its N-terminal end, and comprises a sequence chosen among (*G-G-G), (*K-G-G) and (*A-K-A),
   where * denotes the N-terminal end of the Linker which is covalently bound to Ab.

Ab as used herein refers to an antibody, notably a monoclonal antibody, such as human recombinant monoclonal antibodies, in particular that are specific for cancer antigens, such as CD38, HER2, VEGF, EGFR, etc, so as to target cancer cells. It includes in particular type G immunoglobulines, such as IgG1, IgG2, IgG3 and IgG4. Suitable antibodies in the sense of the invention include bevacizumab, cetuximab, trastuzumab, daratumumab, panitumumab, mabthera.

"Site specific" as used herein refers to the location on the antibody where the attachment of the compound of formula (A) occurs. Typically, this location is Glutamine 295 (Q295) of IgG, where microbial transglutaminase (MTG) is used for the bioconjugation. This corresponds to the reactive glutamine on the heavy chain of different IgG types to be specifically targeted by MTG.

"Oligopeptide" refers to a short chain of less than 15 amino acids linked by peptide (amide) bonds. Typically, an oligopeptide chain has an amino terminal group (or N-terminal end) and a carboxyl (COOH) terminal group or C-terminal end. Thus, according to the invention, the Linker is covalently bound to the antibody (Ab) at its N-terminal end and to the chelator via its C-terminal end.

As used herein, the amino acids may be designated by their 1-letter code, as follows :

| | | | | | |
|---|---|---|---|---|---|
| **G** | Glycine | Gly | **P** | Proline | Pro |
| **A** | Alanine | Ala | **V** | Valine | Val |
| **L** | Leucine | Leu | **I** | Isoleucine | Ile |
| **M** | Methionine | Met | **C** | Cysteine | Cys |
| **F** | Phenylalanine | Phe | **Y** | Tyrosine | Tyr |
| **W** | Tryptophan | Trp | **H** | Histidine | His |
| **K** | Lysine | Lys | **R** | Arginine | Arg |
| **Q** | Glutamine | Glm | **N** | Asparagine | Asn |
| **E** | Glutamic Acid | Glu | **D** | Aspartic Acid | Asp |
| **S** | Serine | Ser | **T** | Threonine | Thr |

According to an embodiment, the Linker is chosen from those of formula: -*G-G-G- (X)p-, -*K-G-G-(X)p-, -*A-K-A-(X)p-

Where * denotes the N-terminal end of the Linker which is covalently bound to Ab.

X is an amino-acid and p is an integer such that 0≤p≤10.

According to an embodiment, said Linker comprises at least the G-G-G sequence. Typically, said Linker may be chosen from -G-G-G- or -G-G-G-G-.

According to a preferred embodiment, said Linker is G-G-G.

CAR refers to the Chelator-to-antibody ratio. It being understood that the compound of formula (I) as defined above may be a mixture of compounds (I) having various CAR. It is generally given as an average value. n thus represents the average CAR (in number) for said mixture.

It may be measured by intact mass analysis, as described by Wong et al. "Precise characterization of intact monoclonal antibodies by the Agilent 6545XT AdvanceBio LC/Q-TF, Application Note, Agilent Technologies, 2017".

According to an embodiment, the process of the invention allows to achieve a CAR comprised between 0.5 and 2, notably between 1 and 2, typically about 1.5 +/- 0.5.

Enzymatic deglycosylation refers to the removal of a glycan moiety of the antibody, typically at the asparagine residue 297 (N297).

According to an embodiment, the enzymatic deglycosylation is carried out with a N-glycosidase.

« N-glycosidase » refers to enzymes that catalyze the cleavage of an internal glycoside bond.

Typically, the N-glycosidase is PNGase F (protein N-glycosidase F from *Flavobacterium meningosepticum).*

PNGase F is commercially available and can be purchased e.g. from ROCHE or R&D SYSTEMS.

The term "Chelator" in the sense of the invention is used to qualify a chemical compound that is able to chelate a metal (ie) to form two or more separate coordinate bonds with a single central metal atom. Such Chelators or chelating agents are typically chemical compounds whose structures permit the attachment of their two or more donor atoms (or sites) to the same metal ion simultaneously and produce one or more rings by chelation.

According to an embodiment, the Chelator is chosen from chelating agents that may chelate radioactive metal isotopes.

According to an embodiment, -Chelator may be of formula (II): 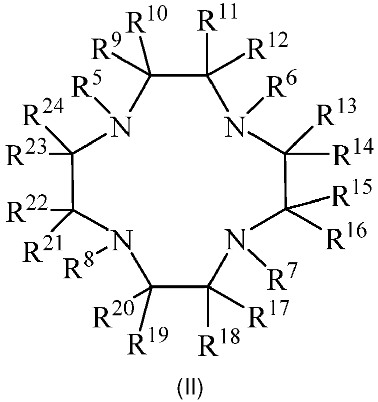 Wherein in Formula (II) :
R⁵, R⁶, and R⁸ are each independently selected from the group consisting of H, (C₁-C₆)alkyl, (C₁-C₆)alkyl-C(=O)-OR²⁵, and (C₁-C₆)alkyl-C(=O)-N (-R²⁵)-R²⁶;
R⁹, R¹⁰, R¹¹, R¹², R¹⁵, R¹⁶, R¹⁷, R¹⁸, R¹⁹, R²⁰ , R²¹, R²², R²³, and R²⁴ are each independently selected from the group consisting of H, (C₁-C₆)alkyl;
R⁷ is independently selected from the group consisting of H, (C₁-C₆)alkyl, (C₁-C₆)alkyl-C(=O)-N(-R²⁵)-R²⁶, and L₁-;
R¹³ and R¹⁴ are each independently selected from the group consisting of H, (C₁-C₆)alkyl, and L¹-;
R²⁵ and R²⁶ are each independently selected from the group consisting of H, (C₁-C₆)alkyl, and (C₁-C₆)alkyl-C(=O)-OH;
Chelator- is bound to Linker- through L¹- ;
Provided one L¹- is present at either R⁷, R¹³ or R¹⁴, with the other R⁷, R¹³ and R¹⁴ being H, (C₁-C₆)alkyl, or (C₁-C₆)alkyl-C(=O)-N(-R²⁵)-R²⁶ respectively, as defined above ;
L¹- is independently selected from a group consisting of
(C₁-C₆)alkyl-C(=O)-NH-(C₁-C₆)alkyl-C(=O)-NH-, (C₁-C₆)alkyl-(C₆H₄)-NH-C(=S)-NH-, C(-CO₂H)-(C₁-C₆)alkyl-(C₆H₄)-NH-C(=S)-NH-, (C₁-C₆)alkyl-C(=O)-NH-, (C₁-C₆)alkyl-C(=O)-NH-(C₁-C₆)alkyl-NH-, (C₁-C₆)alkyl-(C₆H₄)-NH- and (C₁-C₆)alkyl-C(=O)-(O-CH₂-CH₂)₁₋₂₀-C(=O)-NH-.

In particular, in formula (II) :
R⁵, R⁶ and R⁸ represent (C₁-C₆)alkyl-C(=O)-N(-R²⁵)-R²⁶;
R⁹, R¹⁰, R¹¹, R¹², R¹³, R¹⁴, R¹⁵, R¹⁶, R¹⁷, R¹⁸, R¹⁹, R²⁰, R²¹, R²², R²³, R²⁴, R²⁵ and R²⁶ represent H ;
R7 represents L¹ ;
And L¹- represents C₁-C₆)alkyl-C(=O)-NH-(C₁-C₆)alkyl-NH- or (C₁-C₆)alkyl-(C₆H₄)-NH-;

According to an alternative embodiment, the Chelator- derives from the following Chelator precursors :

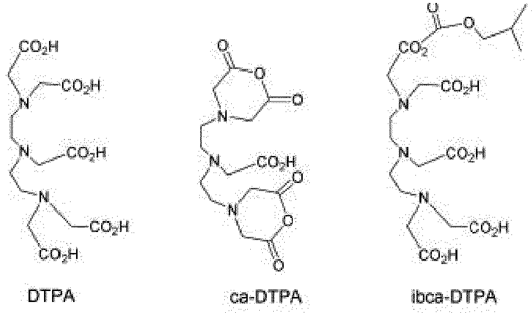

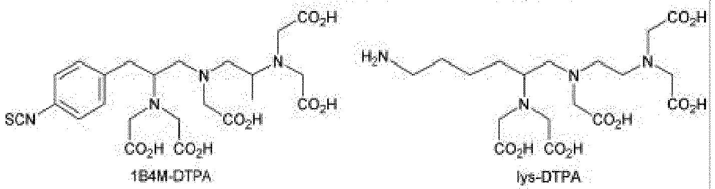

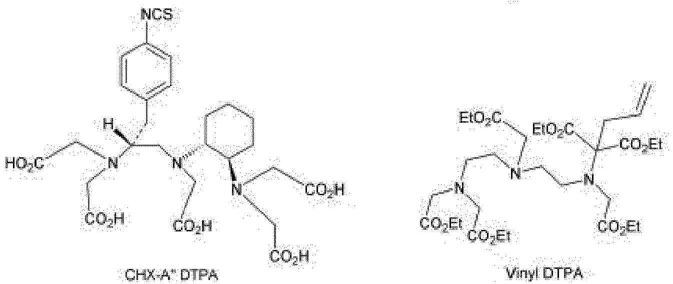

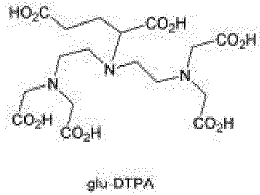

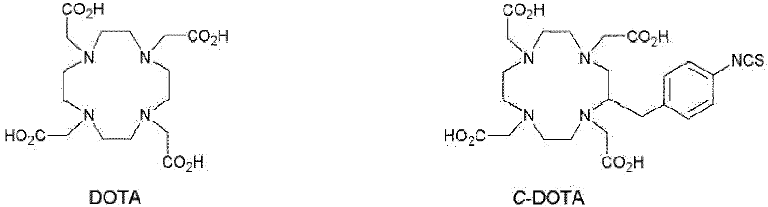

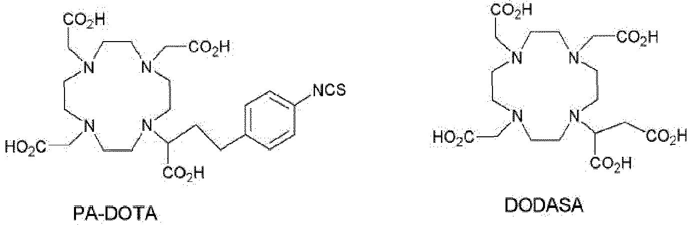

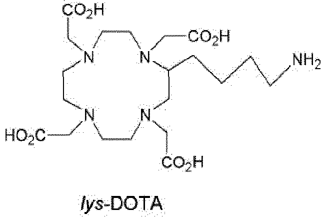

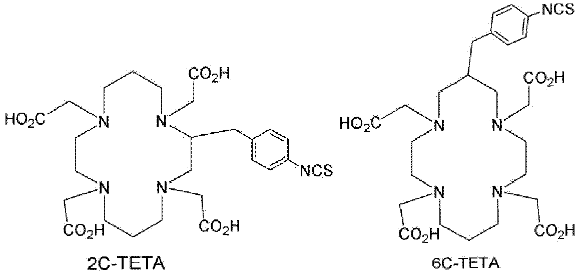

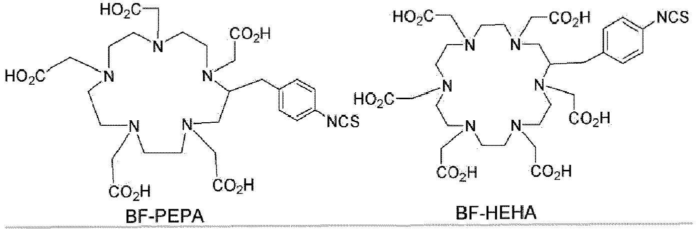

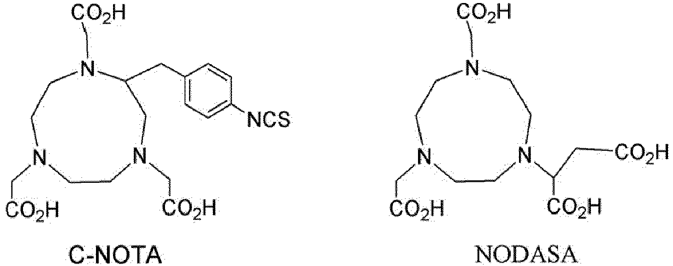

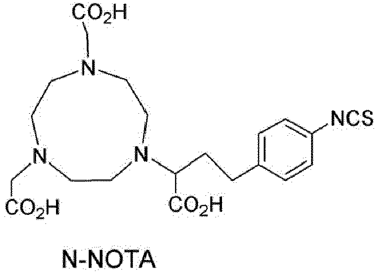

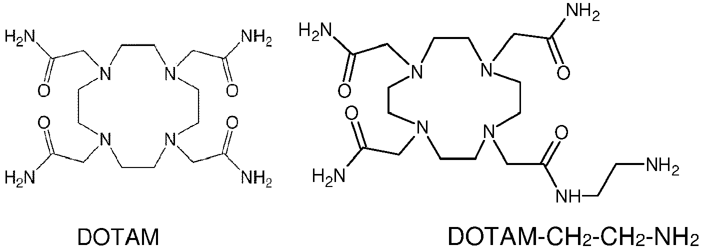

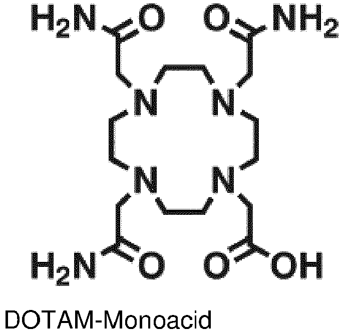

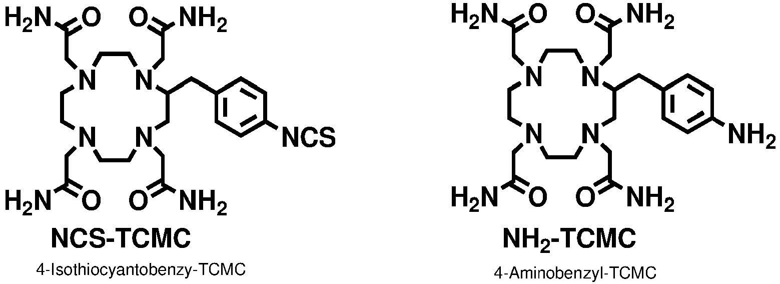

As used above, Chelator precursor is defined as a compound which is suitable to form the compound of formula (A) above when coupled with Linker.

Typically, the Chelator precursor is DOTA, DOTAM, DOTAM-CH₂-CH₂-NH₂ NCS-TCMC or NH₂-TCMC, more preferably NH₂-TCMC.

The Metals as used herein typically refers to radioactive isotopes of metal, that may be useful in nuclear medicine. Such radionuclides include: ⁶⁶Ga, ⁶⁸Ga, ⁸⁶Y, ⁹⁰Y, ¹¹¹In, ²¹²Pb, ²¹²Bi, ²¹³Bi, ⁸⁹Zr, ¹⁷⁷Lu, ²²⁵Ac, and more preferably ²¹²Pb (Lead-212).

"Transglutaminase" refers to an enzyme that may catalyze the formation of an isopeptide bond between γ-carboxamide groups (-(C=O)NH₂) of glutamine residue side chains and the ε-amino groups ( -NH₂) of lysine residue side chains with subsequent release of ammonia (NH₃).

According to an embodiment, the transglutaminase is a microbial transglutaminase. Typically, the transglutaminase may be a transglutaminase MTGase enzyme from the species *Streptomyces mobaraensis.*

Such transglutaminases are commercially available, e.g. MTGase enzyme marketed by ZEDIRA.

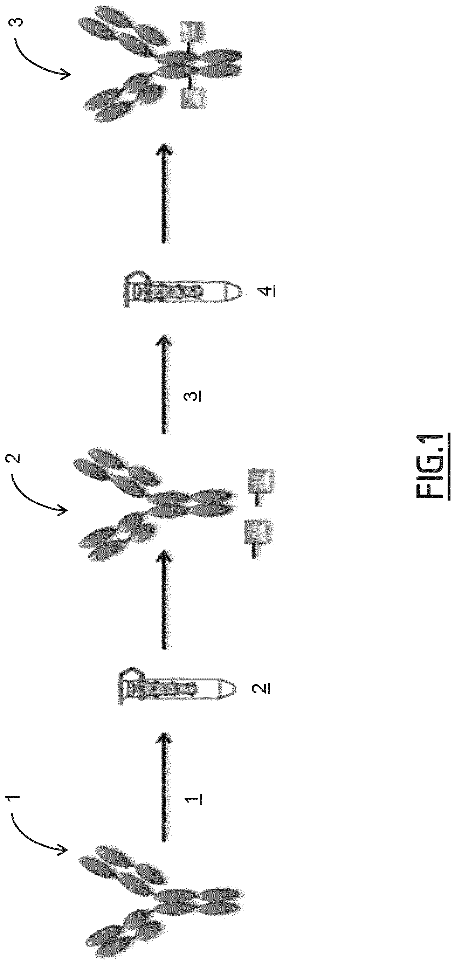

According to the invention, the process includes the step of deglycosylating the antibody with N-glycosidase F (PNGase F). This enzyme works by cleaving N-linked oligosaccharides between innermost GlcNAc and asparagine residues from N-linked glycoproteins, as shown in Figure 1.

According to an embodiment, this leads to the removal of the glycan moiety at the asparagine residue 297 (N297).

The deglycosylation reaction may be usually carried out at a temperature between 20 to 40°C, typically about 23°C to 37°C and between 1 and 24 hours, typically about 15 to 24 hours. The amount of N-glycosidase which is used may be comprised between 100 ng/mg to 1 mg/mg, typically about 500 ng/mg.

Once the antibody is deglycosylated, a diafiltration step may be done to remove excess N-glycosidase F enzyme, following the deglycosylation step.

According to an embodiment, the process of the invention, thus includes a diafiltration step after conducting the deglycosylation step.

Then, the Linker-Chelator (A) compound is reacted with the microbial transglutaminase (MTGase), which will conjugate the compound of formula (A) to the antibody through the Linker free N-Terminal end. This may be done by incubating the compound of formula (A) with the transglutaminase.

Specifically, microbial transglutaminase works by catalyzing the formation of a covalent bond between the γ-carbonyl amide group of glutamines with primary amines of amino acids such as lysine although other amide donors have also been used (Dennler 2014, Mehta 2005). In 2010, Jeger *et al.* showed that a conserved glutamine at position 295 in the heavy chain (constant domain) of deglycosylated IgG₁ antibody's is the only location that MTGases will react, yielding a theoretical Chelator-to-antibody ratio of 2 (one on each heavy chain of the antibody). The enzymatic reaction can be operated with the following conditions:
Temperature: between 20 and 40°C, typically between 23 and 37°C
Amount of transglutaminase (MTGase): between 1 and 10 U/mL, typically about 6U/mL
Time: between 1 to 24 hours, typically between 16 to 24 hours.

Accordingly, this ensures the bioconjugation of the invention is site-specific as the coupling only occurs at this position of the antibody and the obtained bioconjugated compound or mixture of bioconjugated compounds is homogenous, as far as the site of the coupling occurs.

This bioconjugation leads to the formation of the corresponding bioconjugated antibody Ab-(Linker-Chelator)ₙ .

A mixture of compounds with various CAR (ie n) may be obtained. The CAR (n) used herein therefore refers to the average value (in number).

The obtained product may be purified, typically by centrifugation-dialysis and/or further exchanged into a storage buffer such as ammonium acetate.

The antibody may then be characterized by standard procedures, e.g. to ensure that the intermediate enzymes were sufficiently removed, to assess the antibody size, and/or to determine the Chelator-to-antibody ratio.

The Linker-Chelator (A) compound may be prepared by reacting the Linker in the form of its corresponding precursor OH-Linker-Z (where Z may be a protecting group for amines, such as carboxybenzyl (Cbz)) with the Chelator, in the presence of an ester activator, such as HATU (1-[Bis(dimethylamino)methylene]-1H-1,2,3-triazolo[4,5-b]pyridinium 3-oxide hexafluorophosphate, Hexafluorophosphate Azabenzotriazole Tetramethyl Uronium), along with a base, such as Hünig's base (*N,N-*diisopropylethylamine, DIPEA), or triethylamine, in a solvent such as Dimethylacetamide (DMAc or DMA). The reaction maybe carried out at room temperature.

According to a further object, the present invention also concerns a site-specific bioconjugated antibody of formula (I):

Ab-(Linker-Chelator)ₙ (I)

As defined above, obtainable by the process of the invention.

It also concerns the compound of formula (I):
a site-specific bioconjugated antibody of formula (I):

   Ab-(Linker-Chelator)ₙ (I)
as defined above for use as a contrast agent or a drug in nuclear medicine.

It may typically be used for the diagnosis and/or treatment of cancer.

Another object of the invention is also a pharmaceutical composition, which comprises, as active principle, a compound of formula (I) according to the present invention, or a pharmaceutically acceptable salt or solvate thereof. These pharmaceutical compositions comprise an effective dose of at least one compound according to the invention, and at least one pharmaceutically acceptable excipient.

Said excipients may be chosen according to the pharmaceutical form and the administration route desired, among usual excipients known of one of skill in the art. Typically, the compositions of the invention are suitable for systemic administration, such as parenteral administration, such as by the intraperitoneal, intramuscular or intra-venous route.

According to usual practice, the dosage suitable to each patient is determined by the physician according to the administration route, the weight and response of the patient. Typically, the active principle of formula (I) above, its salt or solvate can be administered as a unitary dosage form, in blend with usual pharmaceutical excipients.

The present invention, according to another of its aspects, also relates to a method for the diagnosis and/or treatment of cancer, which comprises the administration to a patient of an effective dose of a compound of formula (I) according to the invention, or a salt with a pharmaceutically acceptable salt thereof.

The present invention is further illustrated by representative Figures and experimental results.

### Figures:

[Fig. 1] Figure 1 is a schematic representation of an illustrative process of the invention, where (1) represents a full length antibody, which is :
- in step 1 deglycosylated,
- in step 2: washed by diafiltration,
- in step 3 : reacted with (2): Linker-Chelator and MTGase
- in step 4: washed by diaflitration,
- to lead to the corresponding bioconjugated antibody (3)

### Experimental Results

### Definitions

PBS- phosphate buffered saline
EDTA - ethylenediaminetetraacetic acid
MWCO - molecular weight cut off
CAR- Chelator-to-antibody ratio
HATU : (1-[Bis(dimethylamino)methylene]-1H-1,2,3-triazolo[4,5-b]pyridinium 3-oxide hexafluorophosphate, Hexafluorophosphate Azabenzotriazole Tetramethyl Uronium)
DIPEA : *N,N*-diisopropylethylamine
DMA : Dimethylacetamide (CH₃C(O)N(CH₃)₂).

### Materials:

NH₂-TCMC (compound 1), was provided by Macrocyclics and used as the principal starting material in the synthesis of GGG-TCMC. All solvents and reagents were purchased from commercial sources and used as received.

### Synthesis of GGG-TCMC :

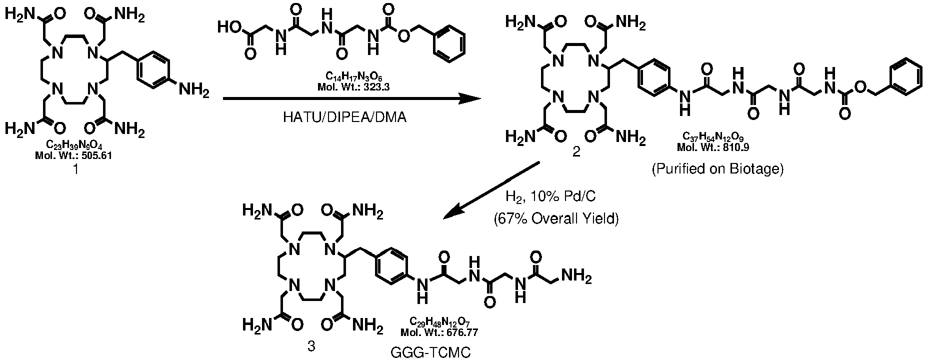

A solution of Z-(Gly)₃-OH (3.3g, 10.2 mmol) of formula: 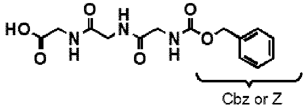 in DMA (64 mL) was prepared followed by the addition of DIPEA (5.20 mL, 29.9 mmol) and HATU (4.51 g, 11.9 mmol).

A solution of NH₂-TCMC (1) (6.56 g, 10.1 mmol) in DMA (80 mL) with DIPEA (5.80 mL, 33.3 mmol) was prepared and added to the prepared peptide solution. The reaction was stirred at room temperature for 17 hours. HPLC monitoring indicated the reaction was incomplete. A second equivalent of Z-(Gly)₃-OH (3.35 g, 10.4 mmol) dissolved in DMA (36 mL) with DIPEA (5.20 mL, 29.9 mmol) and HATU (4.53 g, 11.9 mmol) was added to the reactor and stirred for an additional 22 hours. The reaction solution was added drop-wise to diethyl ether (1.50 L) to precipitate the crude material. Additional diethyl ether (1.0 L) was added to the vessel and stirred for 20 minutes. The precipitate was isolated by vacuum filtration and the filter cake washed with diethyl ether (150 mL). A stock solution was prepared with acetonitrile (120 mL), H₂O (480 mL), and TFA (6 mL). The crude material was purified by reverse phase chromatography using 0.1% TFA in acetonitrile and 0.1% TFA in H₂O mobile phase. Purified fractions of 2 were concentrated by rotary evaporation then transferred to a Parr shaker bottle. 10% Palladium on Carbon catalyst was added and the mixture subjected to vacuum then pressurized to 30 psi hydrogen. Hydrogenation batches were shaken at room temperature until HPLC monitoring indicated reaction completion. Reaction mixture was filtered using 0.2 µm PVDF membrane filter under vacuum and lyophilized to obtain **3** as a solid.

### IgG1 monoclonal antibody targeting CD38 Bioconjugation

This antibody was diafiltered into PBS prior to bioconjugation. Antibody was deglycosylated by adding 6U/mg of PNGase F enzyme and stirring at 37ºC overnight. Enzyme was removed and buffer exchanged to PBS + 50mM EDTA by diafiltration through a 50kDa MWCO filter. Antibody was incubated with 80 mol equivalent of chemical Linker-Chelator (GGG-TCMC) and 6U/ml of MTGase enzyme (Zedira) overnight at 37ºC with gently stirring. Buffer exchange and enzyme removal were performed by diafiltration into 150mM ammonium acetate, pH 4.5.

A final antibody concentration of 6.4mg/ml was determined. CAR was determined by intact mass analysis to be 0.8.

**[Table 1]**

| **Chelators Per Antibody** | **Abundance** |
|---|---|
| 0 | 292,483 |
| 1 | 825,940 |
| 2 | 61,925 |
| **TOTAL** | 1180348 |
| **CAR** | **0.8** |

| | |
|---|---|
| **CAR** = **((825,940) + (2*61.925)) / (1180348)** | |

### Monoclonal IgG1 antibody targeting TEM-1 Bioconjugation

This antibody was diafiltered into PBS before starting bioconjugation. Antibody was deglycosylated by adding 6U/mg of PNGase F enzyme and stirring at 37ºC overnight. Enzyme was removed and buffer exchanged to PBS + 50mM EDTA by diafiltration through a 50kDa MWCO filter. Antibody was incubated with 80 mol equivalent of chemical Linker-Chelator (GGG-TCMC) and 6U/ml of MTGase enzyme (Zedira) overnight at 37ºC with gently stirring. Buffer exchange and enzyme removal were performed by diafiltration into 150mM ammonium acetate, pH 4.5.

A final antibody concentration of 6.5mg/ml was determined. CAR determined by intact mass is 1.2

**[Table 2]**

| **Chelators Per Antibody** | **Abundance** |
|---|---|
| 0 | 161,429 |
| 1 | 1,752,176 |
| 2 | 591,348 |
| **TOTAL** | 2,504,953 |
| **CAR** | **1.2** |

### IgG1 Antibody Bioconjugation

**IgG1** antibody was diafiltered into PBS before starting bioconjugation.

Antibody was deglycosylated by adding 6U/mg of PNGase F enzyme and stirring at 37ºC overnight. Enzyme was removed and buffer exchanged to PBS + 50mM EDTA by diafiltration through a 50kDa MWCO filter. Antibody was incubated with 80 mol equivalent of chemical Linker-Chelator (GGG-TCMC) and 6U/ml of MTGase enzyme (purchased from Zedira) overnight at 37ºC with gently stirring. Buffer exchange and enzyme removal were performed by diafiltration into 150mM ammonium acetate, pH 4.5.

A final antibody concentration of 5.1mg/ml was determined. CAR determined by intact mass analysis is 1.1.

**[Table 3]**

| **Chelators Per Antibody** | **Abundance** |
|---|---|
| 0 | 672,786 |
| 1 | 4,854,318 |
| 2 | 1,258,650 |
| **TOTAL** | 6,785,754 |
| **CAR** | **1.1** |

### Monoclonal IgG1 antibody targeting CD20 Bioconjugation

This antibody was diafiltered into PBS before starting bioconjugation. Antibody was deglycosylated by adding 6U/mg of PNGase F enzyme and stirring at 37ºC overnight. Enzyme was removed and buffer exchanged to PBS + 50mM EDTA by diafiltration through a 50kDa MWCO filter. Antibody was incubated with 80 mol equivalent of chemical Linker-Chelator (GGG-TCMC) and 6U/ml of MTGase enzyme (Zedira) overnight at 37ºC with gently stirring. Buffer exchange and enzyme removal were performed by diafiltration into 150mM ammonium acetate, pH 4.5.

A final antibody concentration of 5.6mg/ml was determined. CAR was determined by intact mass to be 1.0.

**[Table 4]**

| **Chelators Per Antibody** | **Abundance** |
|---|---|
| 0 | 266,076 |
| 1 | 2,257,476 |
| 2 | 233,893 |
| **TOTAL** | 2,757,445 |
| **CAR** | **1.0** |

### Monoclonal antibody targeting HER2 Bioconjugation

This antibody was diafiltered into PBS before starting bioconjugation.

Antibody was deglycosylated by adding 6U/mg of PNGase F (Roche) enzyme and stirring at 37ºC overnight.

Enzyme was removed and buffer exchanged to PBS + 50mM EDTA by diafiltration through a 50kDa MWCO filter.

Antibody was incubated with 80 mol equivalent of chemical Linker-Chelator (GGG-TCMC) and 6U/ml of MTGase enzyme (Zedira) overnight at 37ºC with gently stirring.

Buffer exchange and enzyme removal were performed by diafiltration into 150mM ammonium acetate, pH 4.5.

A final antibody concentration of 2.5mg/ml was determined. CAR was determined by intact mass to be 0.9.

**[Table 5]**

| **Chelators Per Antibody** | **Abundance** |
|---|---|
| 0 | 200,359 |
| 1 | 731,247 |
| 2 | 96,722 |
| **TOTAL** | 1,028,328 |
| **CAR** | **0.9** |

### Monoclonal IgG1 Antibody targeting EGFR Bioconjugation

Antibody was diafiltered into PBS before starting bioconjugation. Antibody was deglycosylated by adding 6U/mg of PNGase F (Roche) enzyme and stirring at 37ºC overnight. Enzyme was removed and buffer exchanged to PBS + 50mM EDTA by diafiltration through a 50kDa MWCO filter. Antibody was incubated with 80 mol equivalent of chemical Linker-Chelator (GGG-TCMC) and 6U/ml of MTGase enzyme (Zedira) overnight at 37ºC with gently stirring. Buffer exchange and enzyme removal performed by diafiltration into 150mM ammonium acetate, pH 4.5.

A final antibody concentration of 3.0mg/ml was determined. CAR was determined by intact mass analysis to be 1.1.

### Monoclonal Antibody Targeting HER2 Bioconjugation - Comparison KGG and GGG Linkers

Lyophilized antibody was resuspended and diafiltered into PBS before starting bioconjugation. Antibody was deglycosylated by adding 500ng/mg of PNGase F enzyme (R&D systems) and stirring at 37ºC overnight. Enzyme was removed by diafiltration through a 50kDa MWCO filter. Antibody was incubated with 80 mol equivalent of chemical linker GGG-TCMC or KGG-TCMC and 6U/ml of MTGase enzyme (Zedira) overnight at 37ºC with gently stirring. Buffer exchange and enzyme removal were performed by diafiltration into 150mM ammonium acetate, pH 4.5.

A final antibody concentration of 5.1mg/ml was determined for the bioconjugation with GGG- Linker and 7.2mg/ml for the bioconjugation with KGG-linker. CAR was determined by intact mass to be 0.8 for KGG-TCMC-Antibody and 1.9 with GGG-TCMC-Antibody.

### Monoclonal IgG1 Antibody Targeting HER2 Antibody Bioconjugation of AKA-TCMC and GGG-TCMC with and without PNGase deglycosylation

This experiment aims at finding whether a PNGase deglycosylation is required to conjugate Chelators to antibodies depending on the linker used. AKA-TCMC was selected as it should not have required PNGase. AKA-TCMC was compared to GGG-TCMC with and without PNGase deglycosylation.

### Procedure

Lyophilized antibody is resuspended and diafiltered into PBS before starting bioconjugation.

Half of the antibody was deglycosylated by adding 500ng/mg of PNGase F enzyme (R&D systems) and rotating at 37ºC overnight. The other half did not receive PNGase F.

Enzyme was removed by diafiltration through a 50kDa MWCO filter and diafiltered into conjugation buffer (PBS+50mM EDTA).

Antibody (deglycosylated and non-deglycosylated) was incubated with 80 mol equivalent of chemical Linker-Chelator (GGG-TCMC) or (AKA-TCMC) and 6U/ml of MTGase enzyme (Zedira) overnight at 37ºC with gentle rotation.

Conjugates were sterile filtered followed by buffer exchange and enzyme removal by diafiltration into 150mM ammonium acetate, pH 4.5. Final sterile filtration performed.

### Results

**[Table 7]**

| | **Final Protein Concentration** | **CAR Value** |
|---|---|---|
| Deglycosylated Ab with GGG-TCMC | 5.7 mg/ml | 1.1 |
| Deglycosylated Ab with AKA-TCMC | 6.7 mg/ml | 0.6 |
| Non-deglycosylated Ab with GGG-TCMC | 6.7 mg/ml | 0 |
| Non-deglycosylated Ab with AKA-TCMC | 7.1 mg/ml | 0 |

The data suggests that deglycosylation is required for AKA-TCMC and GGG-TCMC to be conjugated to an antibody and furthermore, that both AKA- and GGG-TCMC are capable to conjugate to the antibody.

### Monoclonal IgG1 antibody Targeting HER2Bioconjugation of GGG-TCMC and GGGG-TCMC

### Procedure

Lyophilized antibody resuspended and diafiltered into PBS before starting bioconjugation.

Antibody was deglycosylated by adding 500ng/mg of PNGase F enzyme (R&D systems) and rotating at 37ºC overnight.

Enzyme was removed by diafiltration through a 50kDa MWCO filter and diafiltered into conjugation buffer (PBS+50mM EDTA).

Antibody was incubated with 80 mol equivalent of chemical Linker-Chelator (GGG-TCMC) or (GGGG-TCMC) and 2U/ml of MTGase enzyme (Zedira) overnight at 37ºC with gentle rotation.

Conjugates were sterile filtered followed by buffer exchange and enzyme removal by diafiltration into 150mM ammonium acetate, pH 4.5. Final sterile filtration performed.

### Results

**[Table 8]**

| | **Final Protein Concentration** | **CAR Value** |
|---|---|---|
| TCMC-GGG-Antibody | 1.3mg/ml | 0.5 |
| TCMC-GGGG-Antibody | 0.9mg/ml | 0.8 |

## Claims

1. Process for preparing a site-specific bioconjugated antibody of formula (I):
Ab-(Linker-Chelator)ₙ (I)
Where said Linker is an oligopeptide with an N-terminal end,
Chelator is a metal chelating agent,
n is the Chelator-to antibody ratio (CAR), wherein 0<n≤2;
said process comprising :
- the enzymatic deglycosylation of said antibody ;
- the coupling of the obtained deglycosylated antibody with a compound of formula (A):
Linker-Chelator (A)
in the presence of a transglutaminase,
said process being **characterized in that**:
said Linker is bound to Ab at its N-terminal end, and comprises a sequence chosen among (*G-G-G), (*K-G-G) and (*A-K-A),
where * denotes the N-terminal end of the Linker which is covalently bound to Ab.

2. The process according to claim 1 wherein the Linker is chosen from those of formula: -*G-G-G-(X)p-, -*K-G-G-(X)p-, -*A-K-A-(X)p-
Where * denotes the N-terminal end of the Linker, X is an amino-acid and p is an integer such that 0≤p≤10.

3. The process according to claim 1 or 2 wherein said Linker comprises at least the *G-G-G sequence.

4. The process according to anyone of the preceding claims wherein said Linker is *G-G-G or *G-G-G-G.

5. The process according to anyone of the preceding claims wherein said Linker is *G-G-G.

6. The process according to anyone of the preceding claims wherein the enzymatic deglycosylation is carried out with a N-glycosidase.

7. The process according to anyone of the preceding claims wherein the N-glycosidase is PNGase F (protein N-glycosidase F from *Flavobacterium meningosepticum*)*.*

8. The process according to anyone of the preceding claims wherein -Chelator is - NH-CH₂-CH₂-DOTAM or -NH-TCMC.

9. The process according to anyone of the preceding claims wherein the transglutaminase is a microbial transglutaminase.

10. The process according to anyone of the preceding claims wherein the transglutaminase is transglutaminase from the species *Streptomyces mobaraensis.*

11. The process according to anyone of the preceding claims wherein CAR is comprised between 1 and 2.

12. The process according to anyone of the preceding claims wherein a diafiltration wash is carried out between the deglycosylation step and the coupling step.

13. A site-specific bioconjugated antibody of formula (I):
Ab-(Linker-Chelator)ₙ (I)
Where Ab, Linker and Chelator are defined as in anyone of the preceding claims obtainable by the process according to anyone of the preceding claims.

14. A pharmaceutical composition comprising a site-specific bioconjugated antibody of formula (I):
Ab-(Linker-Chelator)ₙ (I)
Where Ab, Linker and Chelator are defined as in anyone of claims 1 to 13, and at least one pharmaceutically acceptable excipient.

15. A site-specific bioconjugated antibody of formula (I):
Ab-(Linker-Chelator)ₙ (I)
Where Ab, Linker and Chelator are defined as in anyone of claims 1 to 13, for use as a contrast agent or a drug in nuclear medicine.

16. The site-specific bioconjugated antibody for use according to claim 14 for diagnosing and/or treating cancer.

## Patentansprüche

1. Verfahren zum Herstellen eines stellenspezifischen biokonjugierten Antikörpers von Formel (I):
Ab-(Linker-Chelator)ₙ, (I)
wobei der Linker ein Oligopeptid mit einem N-terminalen Ende ist,
Chelator ein Metallchelatbildner ist,
n das Chelator-Antikörper-Verhältnis (CAR) ist, wobei 0<n≤2;
das Verfahren umfassend:
- die enzymatische Deglykosylierung des Antikörpers;
- das Koppeln des erlangten deglycosylierten Antikörpers mit einer Verbindung von Formel (A):
Linker-Chelator (A)
in Anwesenheit einer Transglutaminase,
wobei das Verfahren **dadurch gekennzeichnet ist, dass**:
der Linker an seinem N-terminalen Ende an Ab gebunden ist und eine
Sequenz umfasst, die aus (*G-G-G), (*K-G-G) und (*A-K-A) ausgewählt ist, wobei * das N-terminale Ende des Linkers bezeichnet, das kovalent an Ab gebunden ist.

2. Verfahren nach Anspruch 1, wobei der Linker ausgewählt ist aus jenen von Formel: -*G-G-G-(X)p-, -*K-G-G-(X)p-, -*A-K-A-(X)p-,
wobei * das N-terminale Ende des Linkers bezeichnet, X eine Aminosäure ist und p eine ganze Zahl ist, sodass 0≤p≤10.

3. Verfahren nach Anspruch 1 oder 2, wobei der Linker mindestens die Sequenz *G-G-G umfasst.

4. Verfahren nach einem der vorherigen Ansprüche, wobei der Linker *G-G-G oder *G-G-G-G ist.

5. Verfahren nach einem der vorherigen Ansprüche, wobei der Linker *G-G-G ist.

6. Verfahren nach einem der vorherigen Ansprüche, wobei die enzymatische Deglycosylierung mit einer N-Glycosidase durchgeführt wird.

7. Verfahren nach einem der vorherigen Ansprüche, wobei die N-Glycosidase PNGase F (Protein-N-Glycosidase F aus *Flavobacterium meningosepticum*) ist.

8. Verfahren nach einem der vorherigen Ansprüche, wobei -Chelator - NH-CH2-CH2-DOTAM oder -NH-TCMC ist.

9. Verfahren nach einem der vorherigen Ansprüche, wobei die Transglutaminase eine mikrobielle Transglutaminase ist.

10. Verfahren nach einem der vorherigen Ansprüche, wobei die Transglutaminase Transglutaminase von der Spezies *Streptomyces mobaraensis* ist.

11. Verfahren nach einem der vorherigen Ansprüche, wobei CAR zwischen 1 und 2 ist.

12. Verfahren nach einem der vorherigen Ansprüche, wobei zwischen dem Deglykosylierungsschritt und dem Kopplungsschritt eine Diafiltrationswäsche durchgeführt wird.

13. Stellenspezifischer biokonjugierter Antikörper von Formel (I):
Ab-(Linker-Chelator)ₙ, (I)
wobei Ab, Linker und Chelator wie definiert in einem der vorherigen Ansprüche sind, und durch das Verfahren nach einem der vorherigen Ansprüche erlangt werden können.

14. Pharmazeutische Zusammensetzung, umfassend einen stellenspezifischen biokonjugierten Antikörper von Formel (I):
Ab-(Linker-Chelator)ₙ, (I)
wobei Ab, Linker und Chelator wie definiert in einem der Ansprüche 1 bis 13 sind, und mindestens ein pharmazeutisch annehmbarer Hilfsstoff.

15. Stellenspezifischer biokonjugierter Antikörper von Formel (I):
Ab-(Linker-Chelator)ₙ, (I)
wobei Ab, Linker und Chelator wie definiert in einem der Ansprüche 1 bis 13 sind,
zur Verwendung als Kontrastmittel oder als Arzneimittel in Nuklearmedizin.

16. Stellenspezifischer biokonjugierter Antikörper zur Verwendung gemäß Anspruch 14 zur Diagnose und/oder Behandlung von Krebs.

## Revendications

1. Procédé de préparation d'un anticorps bioconjugué spécifique de site de formule (I) :
Ab-(Lieur-Chélateur)ₙ (I)
dans lequel ledit lieur est un oligopeptide avec une extrémité N-terminale,
Le chélateur est un agent de chélation de métaux,
n est le rapport Chélateur/anticorps (CAR), dans lequel 0<n≤2 ;
le procédé comprenant :
- la déglycosylation enzymatique dudit anticorps ;
- le couplage de l'anticorps déglycosylé obtenu avec un composé de formule (A) :
Lieur-Chélateur (A)
en présence d'une transglutaminase,
ledit procédé étant **caractérisé en ce que** :
ledit Lieur est solidaire d'Ab à son extrémité N-terminale, et comprend une séquence choisie parmi (*G-G-G), (*K-G-G) et (*A-K-A),
dans lequel * désigne l'extrémité N-terminale du Lieur qui est liée de manière covalente à Ab.

2. Procédé selon la revendication 1 lequel le Lieur est choisi parmi ceux de formule : -*G-G-G-(X)p-, -*K-G-G-(X)p-, -*A-K-A-(X)p-
dans lequel * désigne l'extrémité N-terminale du Liant, X est un acide aminé et p est un nombre entier tel que 0≤p≤10.

3. Procédé selon la revendication 1 ou 2, dans lequel ledit Lieur comprend au moins la séquence *G-G-G.

4. Procédé selon l'une quelconque des revendications précédentes, dans lequel ledit Lieur est *G-G-G ou *G-G-G-G.

5. Procédé selon l'une quelconque des revendications précédentes, dans lequel ledit Lieur est *G-G-G.

6. Procédé selon l'une quelconque des revendications précédentes, dans lequel la déglycosylation enzymatique est réalisée avec une N-glycosidase.

7. Procédé selon l'une quelconque des revendications précédentes, dans lequel la N-glycosidase est la PNGase F (protéine N-glycosidase F de *Flavobacterium meningosepticum*)*.*

8. Procédé selon l'une quelconque des revendications précédentes, dans lequel le - Chélateur est -NH-CH2-CH2-DOTAM ou -NH-TCMC.

9. Procédé selon l'une quelconque des revendications précédentes dans lequel la transglutaminase est une transglutaminase microbienne.

10. Procédé selon l'une quelconque des revendications précédentes, dans lequel la transglutaminase est une transglutaminase issue de l'espèce *Streptomyces mobaraensis.*

11. Procédé selon l'une quelconque des revendications précédentes, dans lequel CAR est compris entre 1 et 2.

12. Procédé selon l'une quelconque des revendications précédentes, dans lequel un lavage par diafiltration est réalisé entre l'étape de déglycosylation et l'étape de couplage.

13. Anticorps bioconjugué spécifique de site de formule (I) :
Ab-(Lieur-Chélateur)ₙ (I)
Dans lequel Ab, le Liant et le Chélateur sont définis comme dans l'une quelconque des revendications précédentes, pouvant être obtenus par le procédé selon l'une quelconque des revendications précédentes.

14. Composition pharmaceutique comprenant un anticorps bioconjugué spécifique de site de formule (I) :
Ab-(Lieur-Chélateur)ₙ (I)
où Ab, le Lieur et le Chélateur sont définis comme dans l'une quelconque des revendications 1 à 13, et au moins un excipient pharmaceutiquement acceptable.

15. Anticorps bioconjugué spécifique de site de formule (I) :
Ab-(Lieur-Chélateur)ₙ (I)
dans lequel Ab, le Lieur et le Chélateur sont définis comme dans l'une quelconque des revendications 1 à 13,
pour une utilisation comme agent de contraste ou comme médicament en médecine nucléaire.

16. Anticorps bioconjugué spécifique de site à utiliser selon la revendication 14 pour diagnostiquer et/ou traiter le cancer.
